# EUROPÄISCHE PATENTANMELDUNG

(11) **EP 3 338 729 A1**
(43) Veröffentlichungstag der Anmeldung: **27.06.2018**
(21) Anmeldenummer: 18152380.4
(22) Anmeldetag: 07.11.2008
(51) Int. Cl.: A61C 1/07, A61C 17/20, B06B 1/06, H01L 41/083, A61B 17/32

(54) **VERFAHREN ZUM BETRIEB EINER DENTALEN ULTRASCHALLVORRICHTUNG**

(30) Priorität: 07.11.2007 DE 102007053544
(62) Teilanmeldung aus: 08168636.2
(71) Anmelder: Sirona Dental Systems GmbH, 64625 Bensheim (DE)
(72) Erfinder: Rein, Matthias, 64653 Lorsch (DE)
(74) Vertreter: Sommer, Peter

(57) **Zusammenfassung**

Dentale Ultraschallvorrichtung 1 bestehend aus einem Handstück 5, aus einem eine Mittelachse Z aufweisenden Ultraschallantrieb 2 mit mindestens einer ersten und einer zweiten Piezoelementscheibe 2.1, aus einer mindestens eine Resonanzfrequenz R1 für eine Schwingungsrichtung X in einer Schwingungsebene S aufweisenden Werkzeugaufnahme 3.1 für eine Werkzeugspitze 3 und aus einer Regelungseinheit 4, wobei die Piezoelementscheiben 2.1, 2.2 jeweils aus mindestens zwei mit Bezug zu einer Grenzebene E1, E2 gegenüberliegend angeordneten Piezoelementsegmenten 2.1a, 2.1b, 2.2a, 2.2b besteht, wobei aus der Ansteuerung der ersten Piezoelementscheibe 2.1 die Schwingungsrichtung X erzeugbar ist, wobei zumindest die zweite Piezoelementscheibe 2.2 so angeordnet ist, dass die Grenzebene E2 mit Bezug zur Mittelachse Z versetzt zur Grenzebene E1 liegt und die jeweilige Piezoelementscheibe 2.1, 2.2 mittels der Regelungseinheit 4 getrennt ansteuerbar ist
Ferner betrifft die Erfindung ein Verfahren zum Betrieb einer dentalen Ultraschallvorrichtung 1, bei dem eine von mehreren Piezoelementscheiben 2.1, 2.2 aktiv angesteuert wird.

## Beschreibung

Die Erfindung betrifft eine dentale Ultraschallvorrichtung bestehend aus einem Handstück, aus einem eine Mittelachse Z aufweisenden Ultraschallantrieb mit mindestens einer ersten Piezoelementscheibe und mindestens einer zweiten Piezoelementscheibe, aus einer von dem Ultraschallantrieb angetriebenen, mindestens eine Resonanzfrequenz R1 für eine Schwingungsrichtung X rechtwinklig zur Mittelachse Z in einer Schwingungsebene S aufweisenden Werkzeugaufnahme für eine Werkzeugspitze und aus einer Regelungseinheit, die zur Ansteuerung mindestens einer der Piezoelementscheiben dient, wobei die Piezoelementscheiben mit Bezug zu der Mittelachse Z hintereinander angeordnet sind und die Piezoelementscheiben jeweils aus mindestens zwei mit Bezug zu einer Grenzebene E1, E2 gegenüberliegend angeordneten Piezoelementsegmenten bestehen, wobei aus der Ansteuerung der ersten Piezoelementscheibe die Schwingungsrichtung X erzeugbar ist.

Die Erfindung betrifft weiterhin ein Verfahren zum Betrieb einer dentalen Ultraschallvorrichtung.

### Stand der Technik

Die DE 600 01 416 T2 offenbart eine Kontrollvorrichtung für ein zahnärztliches Ultraschallhandstück, die einen piezoelektrischen Transducer eines Schwingungserzeugers des Handstücks regelt. Die Vorrichtung ist in der Lage, die Serienresonanzfrequenz unabhängig von der Art des Gewebes, an welches das Instrument herangeführt wird, zu halten. Dafür sind zwei Stromkreise vorgesehen, ein Arbeitsstromkreis und ein Steuerstromkreis, wobei der Steuerstromkreis die Phasenverschiebung zwischen Strom und Spannung misst und elektrisch kompensiert.

Die DE 10 2005 044 074 A1 beschreibt eine Antriebseinheit zum im Wesentlichen geradlinigen Antreiben eines Werkzeuges mit hoher Frequenz, welche ein Volumen aus piezoelektrischem, magnetostriktivem Arbeitsmaterial und einen Feldgenerator zum Erzeugen eines elektrischen oder magnetischen Wechselfeldes sowie einen an ein treibendes Ende des Arbeitsvolumens angekoppelten Übertragungskörper aufweist, der mit dem Werkzeug verbindbar ist, wobei das Arbeitsmaterialvolumen zwei Teilvolumina aufweist, die zu beiden Seiten einer Achse angeordnet sind und die Teilvolumina des Arbeitsmaterials so polarisiert und so mit dem Wechselfeld beaufschlagt sind, dass sie gegenphasige Antriebsbewegungen erzeugen. Es gibt demnach zwei Teilvolumina, die eine gemeinsame in der Achse angeordnete Grenzebene aufweisen. Das Arbeitsmaterialvolumen ist gemäß Ausführungsbeispiel aus mehreren Piezoelementscheiben gebildet, die wiederum jeweils aus zwei Piezoelementsegmenten aufgebaut sind, welche mit Bezug zu vorgenannter Ebene benachbart angeordnet sind. Die symmetrische Anordnung aller Teilvolumina bzw. aller Piezoelementsegmente gewährleistet einen Antrieb in Richtung rechtwinklig zur Achse des Handgerätes.

Die Erzeugung einer anderen Bewegungsrichtung ist mit der vorgehend beschriebenen Behandlungseinheit nicht möglich.

Bei Einsatz einer Ultraschall-Werkzeugspitze ist es notwendig, die Ultraschall-Werkzeugspitze derart relativ zum Zahn auszurichten, dass die Schwingungsrichtung der Ultraschallbewegung in tangentialer Richtung zum Zahn verläuft.

Es ist daher die Aufgabe der Erfindung, eine Ultraschallvorrichtung und ein Verfahren zum Betrieb einer dentalen Ultraschallvorrichtung anzugeben, die verschiedene Schwingungsrichtungen gewährleisten.

### Offenbarung der Erfindung

Die Aufgabe der Erfindung wird gelöst durch die im Hauptanspruch offenbarte dentale Ultraschallvorrichtung und das im Nebenanspruch offenbarte Verfahren zum Betrieb der dentalen Ultraschallvorrichtung. Vorteilhafte Ausgestaltungen der Erfindungen sind in den Unteransprüchen wiedergegeben.

Die dentale Ultraschallvorrichtung besteht aus einem Handstück, aus einem eine Mittelachse Z aufweisenden Ultraschallantrieb mit mindestens einer ersten Piezoelementscheibe und mindestens einer zweiten Piezoelementscheibe, aus einer von dem Ultraschallantrieb angetriebenen, mindestens eine Resonanzfrequenz R1 für eine Schwingungsrichtung X in einer Schwingungsebene S aufweisenden Werkzeugaufnahme für eine Werkzeugspitze und aus einer Regelungseinheit, die zur Ansteuerung mindestens einer der Piezoelementscheiben dient, wobei die Piezoelementscheiben mit Bezug zu der Mittelachse Z hintereinander angeordnet sind und aus der Ansteuerung der ersten Piezoelementscheibe die Schwingungsrichtung X erzeugbar ist, wobei zumindest die zweite Piezoelementscheibe aus mindestens zwei mit Bezug zu einer die Mittelachse Z aufweisenden Grenzebene E2 gegenüberliegend angeordneten Piezoelementsegmenten besteht.

Wenn die Bewegungs- bzw. Schwingungsrichtung der Ultraschall-Werkzeugspitze in eine Richtung normal zum Zahn erfolgt, so kommt es zu einer mechanischen Dämpfung der Werkzeugspitze. Damit verbunden ist eine Erhöhung des Verlaufs der elektrischen Impedanz |Z|(f) für die entsprechende Resonanzfrequenz R1, R1' bzw. eine Erhöhung des Phasenwinkels zwischen einem Strom I und einer Spannung U. Die Ermittlung des Verlaufs der Impedanz |Z|(f) bzw. der Phase ϕ(f) erfolgt mittels einer Fourier-Analyse des Frequenzverhaltens wie z. B. DFT, FFT oder Zoom-FFT.

Erfindungsgemäß ist vorgesehen, dass die zweite Piezoelementscheibe so angeordnet ist, dass die Grenzebene E2 mit Bezug zur Umfangsrichtung zur Mittelachse Z verdreht bzw. versetzt zur Grenzebene E1 angeordnet ist und die jeweilige Piezoelementscheibe bzw. das jeweilige Piezoelementsegment mittels der Regelungseinheit getrennt ansteuerbar ist. Dadurch, dass zumindest die zweite Piezoelementscheibe mit Bezug zur Mittelachse Z derart angeordnet ist, dass die Grenzebene E2 versetzt zur Grenzebene E1 angeordnet ist und die jeweilige Piezoelementscheibe bzw. das jeweilige Piezoelementsegment mittels der Regelungseinheit getrennt ansteuerbar ist, ist die Generierung verschiedener Schwingungsrichtungen, also beispielsweise einer Schwingungsrichtung Y, die rechtwinklig zur Schwingungsrichtung X verläuft, möglich.

Hierzu kann es vorteilhaft sein, wenn die Regelungseinheit Mittel aufweist, mittels derer zumindest ein von der mechanischen Dämpfung der aktiv angesteuerten Piezoelementscheibe abhängiger Wert W1B, W2B erfassbar ist. Der Wert W1B ist der Wert der ersten Piezoelementscheibe und der Wert W2B ist der Wert der zweiten Piezoelementscheibe. Als Wert W1B, W2B kommt ein Impedanzwert Z1B, Z2B oder ein Phasenwert ϕ1B, ϕ2B zwischen dem Strom I und der Spannung U unter Belastung in Frage. Die Ermittlung des Wertes W1B, W2B erfolgt im aktiv angesteuerten Zustand der zu messenden Piezoelementscheibe. Wenn die zu messende Piezoelementscheibe gerade nicht aktiv angesteuert ist, wird diese zwecks Messung des Wertes W1B, W2B zumindest kurzzeitig, vorzugsweise mit einem Impuls aktiv angesteuert. Als Piezoelementscheibe wird die Summe aller bezüglich der Richtung der Mittelachse Z in einer Höhe liegenden Piezoelementsegmente bezeichnet. Die so gebildete Piezoelementscheibe erstreckt sich rechtwinklig zur Mittelachse Z. Die Piezoelementsegmente einer Piezoelementscheibe können mit und ohne Abstand zueinander angeordnet sein.

Sobald eine erhöhte Dämpfung der Werkzeugspitze erfolgt, also eine Schwingungsrichtung X normal zum Zahn erzeugt wird, erfolgt ein Wechsel der Piezoelementscheibe und somit ein Wechsel der Antriebsrichtung bzw. Ansteuerung, bei der eine Schwingungsrichtung Y in eine Richtung rechtwinklig zur Schwingungsrichtung X und damit tangential zur Zahnoberfläche erzeugt wird. Damit wechselt die Schwingungsrichtung von einer Richtung normal zum Zahn in eine Richtung tangential zum Zahn, so dass keine Dämpfung bzw. eine wesentlich kleinere Dämpfung auftritt. Eine weitere Vorraussetzung für diese Vorgehensweise ist, dass die Regelungseinheit Mittel aufweist, mittels derer zumindest ein von der mechanischen Dämpfung der aktiv angesteuerten Piezoelementscheibe abhängiger Wert W1B, W2B erfassbar ist.

Vorzugsweise handelt es sich bei diesem Wert W1B, W2B um Impedanzwerte Z1B, Z2B. Es ist aber auch möglich, Werte der Phase ϕ heranzuziehen. Der Index "1" bezieht sich auf die jeweilige Piezoelementscheibe. Der Index "B" bezeichnet den Zustand der Belastung. Der Index "L" bezeichnet den Zustand des Leerlaufs. Durch Vergleich der Dämpfung in X- und Y-Richtung, also des Wertes W1B, W2B der jeweiligen Piezoelementscheibe bzw. des jeweiligen Piezoelementsegments, kann auf den Schwingungszustand der Werkzeugspitze geschlossen werden und somit eine Auswahl der gewünschten Schwingungsrichtung bzw. der anzusteuernden Piezoelementscheibe getroffen werden. Somit kann die Bewegungs- bzw. Schwingungsrichtung derart erzeugt werden, dass der tangential zum Zahn verlaufende Bewegungsanteil der Ultraschall-Werkzeugspitze zumindest überwiegt, unabhängig davon, wie der Anwender die Werkzeugspitze relativ zum Zahn positioniert.

Von Vorteil kann es sein, wenn die jeweilige Grenzebene E1, E2 parallel zu der Mittelachse Z angeordnet ist und die Grenzebene E1 mit Bezug zu einer Richtung um die Mittelachse Z um einen Winkel α versetzt bzw. verdreht zu der Grenzebene E2 der zweiten Piezoelementscheibe angeordnet ist. Durch den Einsatz von mehreren Piezoelementscheiben, deren Grenzebenen E1, E2 relativ zueinander versetzt angeordnet sind, können beliebige Schwingungsrichtungen X, Y erzeugt und aktiv unterstützt werden.

Hierzu kann es von Vorteil sein, wenn der Winkel α zwischen 5° und 90° groß ist. Vorzugsweise kann ein Versatz von 90° vorgesehen sein, so dass die Ebene E2 parallel zur Schwingungsebene S angeordnet ist. Somit können zwei rechtwinklig zueinander verlaufende Bewegungsrichtungen X, Y erzeugt werden, die beide eine Pendelbewegung der Werkzeugspitze mit sich bringen. Bei kleinerem Versatz können entsprechend viele Bewegungsrichtungen erzeugt werden, womit die erfindungsgemäße Ultraschallvorrichtung unter Berücksichtigung der jeweiligen Dämpfung entsprechend sensibel ausgebildet werden kann.

Zudem kann es vorteilhaft sein, wenn die Mittel zur Ermittlung oder Speicherung von zumindest einem Wert W1L, W2L zumindest einer Piezoelementscheibe (2.1, 2.2) im Leerlauf ausgebildet sind. Der Index "L" bezeichnet den Leerlaufbetrieb, also den Betrieb der Ultraschallvorrichtung für die jeweilige Schwingungsrichtung ohne Last. Der Wert W1L, W2L dient als Vergleichsgröße zu dem erfindungsgemäß erfassbaren Wert W1B, W2B unter Belastung. Das Kriterium für die Auswahl der Bewegungsrichtung könnte alternativ auch anhand einer von der mechanischen Dämpfung abhängigen absoluten Größe erfolgen. Ein Vergleichswert kann jedoch präziser ermittelt werden.

Zwecks Vergleich kann es vorgesehen sein, dass die Mittel zum Vergleich des Wertes W1B, W2B unter Belastung mit dem jeweiligen Wert W1L, W2L im Leerlauf und zur Ansteuerung derjenigen Piezoelementscheibe ausgebildet sind, bei der eine Abweichung A1, A2 zwischen dem Wert W1B, W2B und dem zugehörigen Wert W1L, W2L kleiner oder am kleinsten ist. Jede der beiden Piezoelementscheiben kann individuell angesteuert werden. Die Abweichung A1 ist die Abweichung des Wertes W1B von dem Wert W1L der ersten Piezoelementscheibe und die Abweichung A2 ist die Abweichung des Wertes W2B von dem Wert W2L der zweiten Piezoelementscheibe ist. Über den Vergleich mit dem jeweiligen Leerlaufverhalten kann unabhängig von anderen Parametern wie beispielsweise die Geometrie der Ultraschallspitze ein optimales Auswahlkriterium zum Bestimmen der Schwingungsrichtung geschaffen werden.

Vorteilhaft kann es sein, wenn in Richtung der Mittelachse Z die Polarisierung der benachbarten Piezoelementsegmente oder die Polarität der elektrischen Ansteuerung der benachbarten Piezoelementsegmente entgegengesetzt ausgebildet ist. Dadurch wird die Grenzebene E1, E2 zwischen den jeweils gegenüberliegenden Piezoelementsegmenten festgelegt. Die Grenzebene E1, E2 definiert nicht unbedingt eine räumliche oder materielle Grenze. Die Grenzebene E1, E2 definiert lediglich die Bereiche unterschiedlicher bzw. entgegengesetzter Polarisierung der Polarität der Piezoelementsegmente bzw. der elektrischen Ansteuerung zwecks Erzeugung von entgegengesetztem Schwingungsverhalten innerhalb einer Piezoelementscheibe. Zwecks Erhalt einer entgegengesetzten Polarisierung der Piezoelementsegmente werden die Piezoelementsegmente, die die jeweilige Piezoelementscheibe bilden, mit Bezug zur Mittelachse Z in umgekehrter Ausrichtung der Polarität angeordnet. Die elektrischen Kontakte bzw. das elektrische Feld kann in diesem Fall für alle Piezoelementsegmente gleich sein, da die Polarisierung des Piezoelements mit Bezug zur Mittelachse Z entgegengesetzt ausgerichtet ist.

Vorteilhaft kann es sein, wenn das jeweilige Piezoelementsegment teilkreisscheibenförmig oder rechteckförmig ausgebildet ist.

Vorteilhaft kann es ferner sein, wenn das Handstück und/oder die Regelungseinheit ein Signalmittel aufweist, über das ein akustisches, optisches und/oder taktiles Signal erzeugbar ist, wenn die mechanische Dämpfung der Werkzeugspitze bzw. die Abweichung A1, A2 einen kritischen Wert überschreitet. Somit ist der Anwender im Fall einer zu hohen Last bzw. einer Überlast rechtzeitig informiert und kann die Bearbeitungskraft reduzieren, bis das System eine angepasste Bewegungsrichtung durch Wechsel der Resonanzfrequenz erreicht hat. Alternativ kann der Anwender durch Veränderung der relativen Position zwischen der Ultraschallspitze und dem Zahn eine Ultraschallbewegung in Richtung normal zur Oberfläche desselben verhindern. Insbesondere ein taktiles Signal im Handstück kann eine schnellstmögliche Erkennung einer Überlast gewährleisten, wobei der Patient durch ein mögliches akustisches Signal nicht irritiert wird.

Die Erfindung betrifft auch ein Verfahren zum Betrieb einer dentalen Ultraschallvorrichtung, bei dem ein von der mechanischen Dämpfung abhängiger Wert W1B, W2B einer aktiv angesteuerten Piezoelementscheibe zumindest für die Resonanzfrequenz R1 bzw. eine Hauptresonanzfrequenz erfasst wird und der Wert W1B, W2B mit zumindest einem für die Resonanzfrequenz R1 und die entsprechende Piezoelementscheibe im Leerlauf ermittelten Wert W1L, W2L verglichen wird, wobei diejenige Piezoelementscheibe aktiv angesteuert wird, bei der die Abweichung A1, A2 zwischen dem Wert W1B, W2B der jeweiligen Piezoelementscheibe unter Belastung und dem Wert W1L, W2L kleiner oder am kleinsten ist. Vorzugsweise handelt es sich bei diesem Wert um Impedanzwerte Z1L, Z2L. Es ist aber auch möglich, den Verlauf des Phasenwinkels ϕ(f) bzw. Phasenwinkelwerte heranzuziehen. Der Index "1" bezieht sich auf die jeweilige Piezoelementscheibe. Der Index "B" bezeichnet den Zustand der Belastung. Der Index "L" bezeichnet den Zustand des Leerlaufs. Durch die Ermittlung der vorhandenen Dämpfung kann auf den Dämpfungszustand geschlossen werden und somit eine Auswahl der gewünschten Schwingungsrichtung X, Y bzw. der anzusteuernden Piezoelementscheibe getroffen werden. Somit ist die Generierung verschiedener Schwingungsrichtungen, also beispielsweise einer Schwingungsrichtung Y möglich, die rechtwinklig zur Schwingungsrichtung X verläuft. Sobald eine erhöhte Dämpfung der Werkzeugspitze 3, also eine Schwingungsrichtung X normal zum Zahn erzeugt wird, kann ein Wechsel der Piezoelementscheibe und somit ein Wechsel der Antriebsrichtung erfolgen, bei der die Schwingungsrichtung Y in Richtung rechtwinklig zur Schwingungsrichtung X und damit tangential zur Zahnoberfläche erzeugt wird. Somit wechselt die Schwingungsrichtung von einer Richtung normal zum Zahn in eine Richtung tangential zum Zahn, so dass keine Dämpfung bzw. eine wesentlich kleinere Dämpfung auftritt. Als Wert W1B, W2B werden vorzugsweise die Impedanzwerte Z1B, Z2B verwendet, die im Fall einer Dämpfung im Verhältnis zum Leerlaufprozess größer sind.

Alternativ kann neben einem Wechsel der Piezoelementscheibe (EIN/AUS) auch eine phasenverschobene Ansteuerung der beiden Piezoelementscheiben, also ein Überblenden der Ansteuerung von X- und Y-Richtung vorgesehen sein.

Hierzu kann der Wert W1B, W2B ein Impedanzwert Z1B, Z2B oder ein Phasenwert ϕ1B, ϕ2B zwischen dem Strom I und der Spannung U unter Belastung und der Wert W1L, W2L ein Impedanzwert Z1L, Z2L oder ein Phasenwert ϕ1L, ϕ2L im Leerlauf sein. Sowohl die Impedanz als auch die Phase ändern sich unter Last.

Dabei kann es von Vorteil sein, wenn bei vorstehender Verfahrensweise zwecks Bewertung der Abweichung A1, A2 ein Quotient Q1, Q2 von dem Impedanzwert Z1B, Z2B zu dem entsprechenden Impedanzwert Z1L, Z2L oder von dem Phasenwert ϕ1L, ϕ2L zu dem entsprechenden Phasenwert ϕ1B, ϕ2B berechnet wird und diejenige Piezoelementscheibe aktiv angesteuert wird bei der der Quotient Q1, Q2 kleiner ist. Vorzugsweise handelt es sich bei dem Wert um die Impedanzwerte der jeweiligen Piezoelementscheibe bzw. des jeweiligen Piezoelementsegments. Der Quotient Q1, Q2 stellt die bevorzugte Größe zur Bewertung der Dämpfung dar. Da die Phase im Belastungsfall im Gegensatz zur Impedanz abfällt, muss bei der Betrachtung des kleinsten Quotienten Q1, Q2 für die Phase der Kehrwert, also der Leerlaufwert zum Belastungswert berücksichtigt werden.

Ein Quotient Q1 für die erste Piezoelementscheibe von beispielsweise dem Impedanzwert Z1B zu dem Impedanzwert Z1L mit einem Wert von eins oder etwas mehr als eins entspricht dem Leerlauf. Sobald der Quotient Q1 einen Wert von mehr als zwei aufweist, entspricht dies einer starken Dämpfung bzw. Belastung, die erfindungsgemäß durch Wechsel der Antriebsrichtung verhindert bzw. reduziert wird. Somit kann die Bewegungs- bzw. Schwingungsrichtung derart erzeugt werden, dass der tangential zum Zahn verlaufende Bewegungsanteil der Ultraschall-Werkzeugspitze zumindest überwiegt, unabhängig davon, wie der Anwender die Werkzeugspitze relativ zum Zahn positioniert. Ein Quotient Q1 für die erste Piezoelementscheibe von beispielsweise dem Impedanzwert Z1B zu dem Impedanzwert Z1L mit einem Wert von eins oder etwas mehr als eins entspricht dem Leerlauf. Sobald der Quotient Q1 einen Wert von mehr als zwei aufweist, entspricht dies einer starken Dämpfung bzw. Belastung, die erfindungsgemäß durch Wechsel der Antriebsrichtung verhindert bzw. reduziert wird.

Es ist auch die Verwendung der Phase ϕ möglich. Die Grenzebene E1, E2 derjenigen Piezoelementscheibe, bei der der Quotient Q1, Q2 kleiner ist, ist um den Winkel α versetzt zu der Grenzebene E1, E2, deren Quotient Q1, Q2 größer ist. Vorzugsweise beträgt der Winkel α 90°. Es ist aber auch eine feinere Auflösung der Schwingungsrichtung durch den Einsatz von Piezoelementscheiben mit kleinerem relativem Winkelversatz der Grenzebenen E1, E2 möglich. Daneben ist auch eine phasenverschobene Ansteuerung der Piezoelementscheiben möglich.

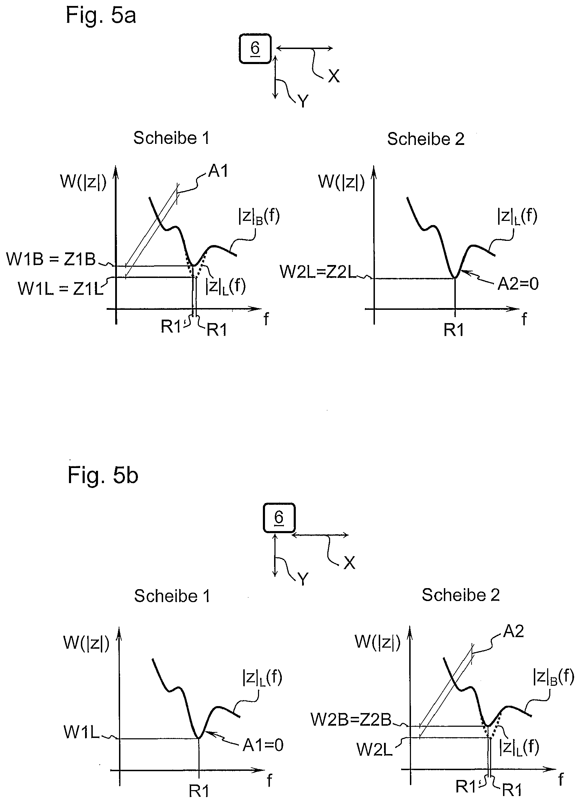

Hierbei kann es vorteilhaft sein, wenn bei Überschreiten eines Schwellenwertes für die Abweichung A1, A2 oder für den Quotienten Q1, Q2 diejenige Piezoelementscheibe aktiv angesteuert wird, deren Grenzebene E2 um den Winkel α versetzt zu der aktuell aktiv angesteuerten Piezoelementscheibe angeordnet ist. Die Schwingungsrichtung X, bei der der größte Quotient (Q1 = Z1B/Z1L für die Impedanz) ermittelt wird (Ausführungsbeispiel Fig. 5a), schließt mit einer Flächennormalen der Zahnoberfläche, an der die Ultraschallspitze anliegt, den kleinsten Winkel ein. Sie hat also den größten Anteil in normaler Richtung, so dass die Dämpfung und damit der Impedanzwert Z1B und der Quotient Q1 am größten sind. Wenn die Schwingungsrichtung X normal zu der Zahnoberfläche verläuft, so ist eine Änderung der Schwingungsrichtung bzw. des Winkels von 90° vorteilhaft, da die Schwingungsrichtung Y dann tangential zum Zahn verläuft.

Gemäß einer Weiterbildung kann der Vergleich des Wertes W1B, W2B mit dem jeweiligen Wert W1L, W2L, das Vergleichen der Abweichung A1, A2 und/oder das Aktivieren der Ansteuerung der Piezoelementscheibe, die die kleinere Abweichung aufweist, mit einer Frequenz zwischen 10 Hz und 500 Hz, insbesondere zwischen 20 Hz und 200 Hz erfolgen. Die Bewertung der Abweichung A1, A2 kann mit einer möglichst hohen Frequenz erfolgen, also einer möglichst hohen Frequenzauflösung, damit die an der Ultraschallspitze anliegende Last mit ausreichender Auflösung überprüft und somit eine Verletzung des Zahns verhindert werden kann.

Vorteilhafterweise die Regelungseinheit ein Warnsignal erzeugen, wenn die mechanische Dämpfung der Werkzeugspitze einen kritischen Wert oder der Quotient Q1, Q2 einen Wert größer als 1,5 vorzugsweise einen Wert von 2 oder 3 überschreitet. Somit kann der Anwender im Fall einer zu hohen Last bzw. einer Überlast rechtzeitig informiert werden und kann die Bearbeitungskraft reduzieren, bis das System eine angepasste Bewegungsrichtung erreicht hat. Alternativ kann der Anwender durch Veränderung der relativen Position zwischen der Ultraschallspitze und dem Zahn eine Ultraschallbewegung in Richtung normal zur Oberfläche desselben verhindern.

Wenn in allen Richtungen eine Dämpfung der Schwingung vorliegt, kann es von Vorteil sein, ein Warnsignal zu erzeugen und gegebenenfalls die Leistung zu verringern.

Ferner kann es vorteilhaft sein, wenn eine Verschiebung zwischen der Resonanzfrequenz R1 im Leerlauf und der Resonanzfrequenz R1' unter Belastung bei Ermittlung des Wertes W1B, W2B und des Wertes W1L, W2L berücksichtigt wird. Der Zahlenwert für die Resonanzfrequenz R1 im Leerlauf und der Zahlenwert für die Resonanzfrequenz R1' im Belastungsfall können sich leicht unterscheiden. Dieser Unterschied wird bei der Ermittlung des jeweiligen Wertes W1B, W2B berücksichtigt. Daneben kann es zwecks Ausnutzung der vollen Leistung der Ultraschallvorrichtung vorgesehen sein, anhand des Impedanzverlaufs |Z|(f) oder des Phasenverlaufs ϕ(f) die genaue Resonanzfrequenzen R1' zu ermitteln und einzustellen. Alternativ kann zwar auf die bereits bekannte Leerlauf-Resonanzfrequenz R1 zurückgegriffen werden, diese kann jedoch um mehrere 100 Hz von dem exakten Wert R1' abweichen.

### Kurzbeschreibung der Zeichnungen

Das erfindungsgemäße Verfahren wird anhand der Zeichnungen erläutert. Es zeigen:
- Fig. 1: eine Prinzipskizze einer erfindungsgemäßen dentalen Ultraschallvorrichtung;
- Fig. 2a: eine Impedanzkurve im Leerlauf;
- Fig. 2b: eine Phasenkurve im Leerlauf;
- Fig. 3: eine vereinfachte Darstellung der Ultraschallvorrichtung mit zwei Piezoelementscheiben;
- Fig. 4a: eine vereinfachte Explosionsdarstellung eines Piezoelementscheiben-Pakets mit vier Piezoelementscheiben;
- Fig. 4b: eine Skizze der Bewegungsrichtung X, Y, Z der Werkzeugspitze;
- Fig. 5a: einen Impedanzverlauf |Z|(f) für die erste Piezoelementscheibe und die zweite Piezoelementscheibe mit Dämpfung in Richtung X;
- Fig. 5b: einen Impedanzverlauf |Z|(f) mit Dämpfung in Richtung Y;
- Fig. 5c: einen Impedanzverlauf |Z|(f) mit Dämpfung in Richtung X und Y;
- Fig. 6: einen Phasenverlauf ϕ(f) für die erste Piezoelementscheibe und die zweite Piezoelementscheibe mit Dämpfung in Richtung X gemäß Fig. 5a.

### Ausführungsform der Erfindung

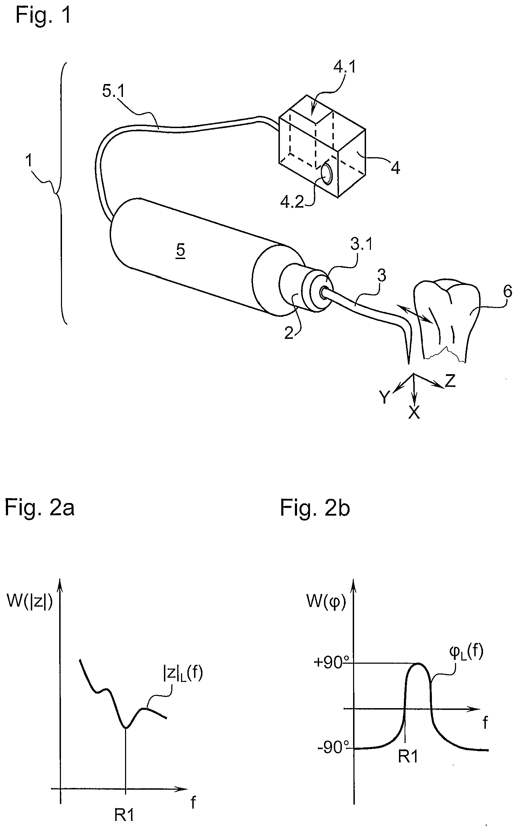

Es zeigt Fig. 1 eine Ultraschallvorrichtung 1 mit einem einen Ultraschallantrieb 2 aufweisenden Handstück 5. An dem Ultraschallantrieb 2 ist stirnseitig eine zweidimensional geformte Werkzeugspitze 3 angeordnet, die an einem Zahn 6 anlegbar ist.

Das Handstück 5 ist über eine Versorgungsleitung 5.1 mit einer Regelungseinheit 4 verbunden. Die Regelungseinheit 4 weist hierbei verschiedene Mittel 4.1 zur Erfassung des Verlaufes des sich während der Belastung der Werkzeugspitze 3 einstellenden Impedanzverlaufs |Z|_{B}(f), Mittel 4.1 zum Vergleich des Impedanzverlaufs |Z|_{B}(f) mit einer im Leerlauf ermittelten Impedanzverlauf |Z|_{L}(f) sowie Mittel 4.1 zur Steuerung des Ultraschallantriebs 2. Ferner weist die Regelungseinheit 4 ein Signalmittel 4.2 auf, welches beim Überschreiten einer an der Werkzeugspitze 3 auftretenden Überlast den Benutzer akustisch und/oder optisch informiert. Das Signalmittel 4.2 kann dabei auch am Handstück 5 angeordnet sein.

Fig. 2a zeigt den Verlauf des Wertes W(|Z|) für die Impedanz |Z|(f) des aktiv angesteuerten Ultraschallantriebs 2 in Abhängigkeit der Anregungsfrequenz f im Leerlauf, also |Z|_{L}(f). Die zweidimensional geformte Werkzeugspitze 3 weist mindestens eine Resonanzfrequenz R1 auf. In der Resonanzfrequenz R1 weist der Impedanzverlauf |Z|_{L}(f) ein Minimum auf. Aus einer Anregung mit der Resonanzfrequenz R1 resultiert eine Schwingungsrichtung X der Werkzeugspitze 3 gemäß Fig. 1.

Fig. 2b zeigt den Verlauf des Wertes W(ϕ) für eine Phase ϕ zwischen einem Strom I und einer Spannung U des aktiv angesteuerten Ultraschallantriebs 2 in Abhängigkeit der Anregungsfrequenz f im Leerlauf. In der Resonanzfrequenz R1 weist der Phasenverlauf ϕ(f) einen Wert von etwa Null auf und steigt auf ein Maximum von ϕ_{L} = 90° an. Die Phase ϕ steigt auf ein Maximum an; dieses Maximum muss nicht 90° sein, es kann auch deutlich darunter liegen.

Bei der Behandlung des Zahnes 6 gemäß Fig. 1 ist es notwendig, dass die Bewegungsrichtung der Werkzeugspitze 3 in eine Richtung tangential zu der im Berührungspunkt zwischen der Werkzeugspitze 3 und dem Zahn 6 befindlichen Zahnoberfläche erfolgt. Gemäß Ausführungsbeispiel Fig. 1 ist das in Richtung X sowie in Richtung Z.

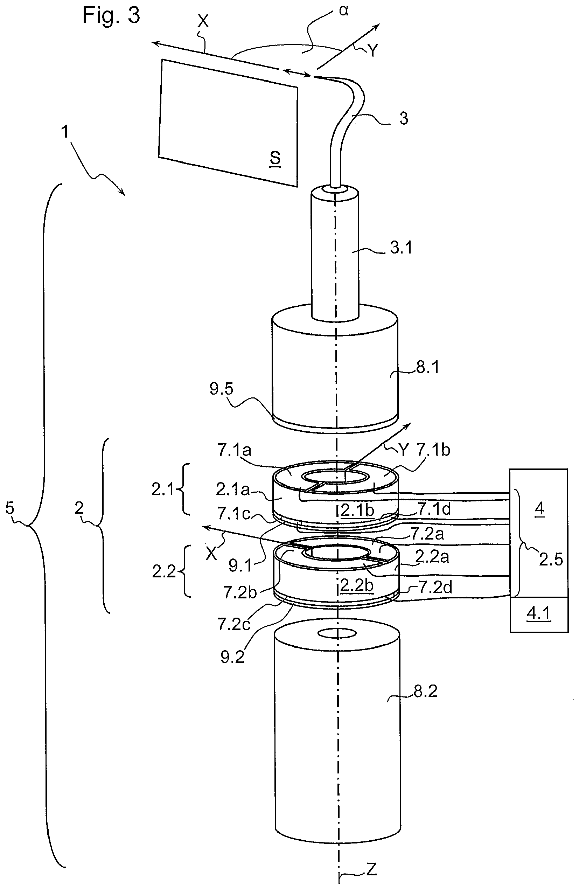

Fig. 3 zeigt eine Explosionsskizze einer Ultraschallvorrichtung 1 mit einem einen Ultraschallantrieb 2 aufweisenden Handstück 5, an dessen stirnseitigem Ende der Werkzeugspitze 3 über eine Werkzeugaufnahme 3.1 angeordnet ist. Das Handstück 5 weist neben dem Ultraschallantrieb 2 eine Schwungmasse 8.1 sowie eine gegenüberliegend zum Antrieb 2 angeordnete Endmasse 8.2 auf.

Der Ultraschallantrieb 2 besteht aus einer ersten Piezoelementscheibe 2.1 und einer koaxial zur ersten Piezoelementscheibe 2.1 und zur Mittelachse Z angeordneten zweiten Piezoelementscheibe 2.2. Die jeweilige Piezoelementscheibe 2.1, 2.2 weist eine einheitliche Polarität auf und ist an der Oberseite über separate Kontaktelemente 7.1a, 7.1b, 7.2a, 7.2b und an der Unterseite über separate Kontaktelemente 7.1c, 7.1d, 7.2c, 7.2d elektrisch kontaktiert. Aufgrund dieser halbkreisscheibenförmigen Kontaktelemente 7.1a-7.2b werden pro Scheibe 2.1, 2.2 jeweils zwei unterschiedlich ansteuerbare Piezoelementsegmente 2.1a, 2.1b, 2.2a, 2.2b gebildet. Die zwei Piezoelementsegmente 2.1a, 2.1b, 2.2a, 2.2b einer Piezoelementscheibe 2.1, 2.2 können somit gleichsinnig oder gegensinnig angesteuert werden. Das jeweilige Kontaktelement 7.1a - 7.2d ist über entsprechende Kontaktleitungen 2.5 mit einer Regelungseinheit 4 elektrisch verbunden. Aufgrund der unterschiedlichen Kontaktierung der jeweils benachbarten Piezoelementsegmente 2.1a, 2.1b, 2.2a, 2.2b einer Piezoelementscheibe 2.1, 2.2 ergibt sich eine unterschiedliche mechanische Bewegung. Während das eine Piezoelementsegment 2.1a kontrahiert, kann sich das benachbarte Piezoelementsegment 2.1b bei unterschiedlicher Kontaktierung ausdehnen.

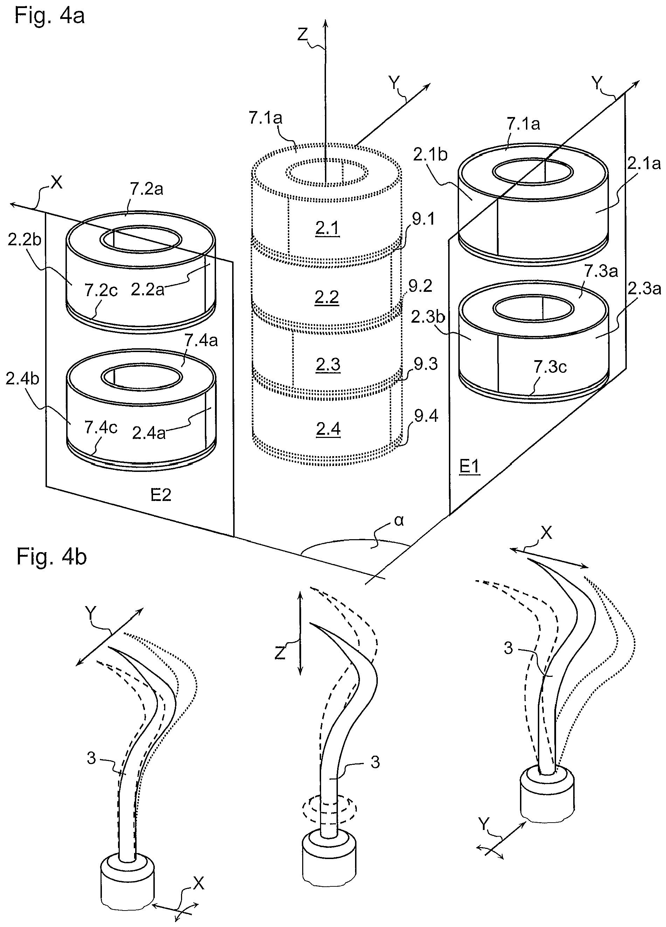

Aufgrund der unterschiedlichen Polarisierung der elektrischen Kontaktierung der Piezoelementsegmente 2.1a - 2.2b ergibt sich eine gemäß Fig. 4a dargestellte, die Bewegung betreffende fiktive Grenzebene E1, E2, die parallel zur jeweiligen Richtungsachse Y, X angeordnet ist.

Bei gleicher elektrischer Kontaktierung ergibt sich eine gleichförmige Bewegung aller Piezoelementsegmente 2.1a-2.2b ohne Grenzebenen. Auf der Unterseite der jeweiligen Piezoelementscheibe 2.1, 2.2 ist zwecks elektrischer Isolation eine Isolierscheibe 9.1, 9.2 vorgesehen. Ergänzend ist auf der Unterseite der Schwungmasse 8.1 eine Isolierscheibe 9.5 vorgesehen.

In der Darstellung gemäß Fig. 4a ist der Piezoelementantrieb bzw. Scheibenstapel 2 alleine bzw. explodiert dargestellt, wobei im Unterschied zu Fig. 3 vier Piezoelementscheiben 2.1 - 2.4 angeordnet sind, wovon jeweils zwei dieselbe Grenzebene E1, E2 aufweisen. Die jeweilige Piezoelementscheibe 2.1 - 2.4 weist unterschiedlich polarisierte Piezoelementsegmente 2.1a - 2.4b auf, deren Polarität gegensinnig ausgerichtet ist. Die jeweiligen Piezoelementsegmente 2.1a - 2.4b sind benachbart angeordnet und weisen in der Regel keinen Abstand auf. Somit ist nur ein gemeinsames oberes Kontaktelement 7.1a - 7.4a und ein gemeinsames unteres Kontaktelement 7.1c-7.4c für die jeweilige Piezoelementscheibe 7.1 - 7.4 notwendig. Aufgrund der Ausrichtung der Polarität entsteht trotz einheitlichen elektrischen Feldes die gegenpolige Bewegung innerhalb einer Piezoelementscheibe 2.1 - 2.4. Die erste Piezoelementscheibe 2.1 und die dritte Piezoelementscheibe 2.3 weisen gemeinsam die Grenzebene E1 auf, welche in Richtung der Achse Y ausgerichtet ist. Die zweite Piezoelementscheibe 2.2 und die vierte Piezoelementscheibe 2.4 weisen die rechtwinklig zur Grenzebene E1 angeordnete Grenzebene E2 auf, die der Richtung X nach ausgerichtet ist. Aus der elektrischen Ansteuerung der jeweiligen Piezoelementscheibe 2.1 - 2.4 ergibt sich für die erste und dritte Piezoelementscheibe 2.1, 2.3 jeweils eine Kippbewegung um die Y-Achse, während bei der zweiten und vierten Piezoelementscheibe 2.2, 2.4 eine Kippbewegung um die X-Achse erzeugt wird. Die Piezoelementscheiben 2.1 - 2.4 sind über Isolierscheiben 9.1 - 9.4 elektrisch isoliert.

Beim Anlegen einer gegenphasigen Wechselspannung an die benachbarten Piezoelementsegmente 2.1a-2.2b ergibt sich für die Schwungmasse 8.1 eine alternierende Kippbewegung um die jeweilige Achse Y, X der jeweiligen Piezoelementscheibe 2.1, 2.2.

In Fig. 4b sind die aus der jeweiligen Kippbewegung entstehenden Schwingungsbewegungen X, Y, Z einer Werkzeugspitze 3 dargestellt. Bei Ansteuerung der zweiten und vierten Piezoelementscheibe 2.2, 2.4 erfolgt also eine Kippbewegung um die X-Achse, was zu eine Schwingungsbewegung der Werkzeugspitze 3 in Richtung der Y-Achse führt. Die Schwingungsrichtung X erfolgt also quer zum auslaufenden Ende der Werkzeugspitze 3.

Bei einer Ansteuerung der ersten und dritten Piezoelementscheibe 2.1, 2.3 erfolgt die Schwingungsbewegung X der Werkzeugspitze 3 rechtwinklig zu der vorgehend beschriebenen Schwingungsrichtung Y in Schwingungsrichtung X. Die Werkzeugspitze 3 pendelt also ausgehend von ihrer Aufnahme 3.1 longitudinal zu ihrem auslaufenden Ende vor und zurück.

Neben dem in den Fig.4a beschriebenen Einsatz entgegengesetzt polarisierter Piezoelementsegmente 2.1a, 2.1b, 2.2a, 2.2b einer Piezoelementscheibe 2.1, 2.2 und einheitlichem Spannungsfeld ist es gemäß Ausführungsform Fig. 3 vorgesehen, für jedes Piezoelementsegment 2.1a, 2.1b, 2.2a, 2.2b eine eigene elektrische Kontaktierung 7.1a - 7.2c bzw. gegenpolige Spannungsfelder benachbarter Piezoelementsegmente 2.1a, 2.1b, 2.2a, 2.2b vorzusehen. Somit kann die jeweilige Piezoelementscheibe 2.1, 2.2 neben dem erfindungsgemäßen gegenpoligen Bewegungszyklus unter Einsatz unidirektionaler Spannungsfelder auch in herkömmlicher Weise unter Einsatz unidirektionaler Spannungsfelder bewegt bzw. angetrieben werden.

Unter Bezug auf die Darstellung des Verlaufs des Wertes W(|Z|) für die Impedanz |Z|(f) gemäß Fig. 2a, 2b zeigt Fig. 5a einen Impedanzverlauf |Z|_{B}(f) bzw. |Z|_{L}(f) für die aktiv angesteuerte erste Piezoelementscheibe 2.1 und die aktiv angesteuerte zweite Piezoelementscheibe 2.2. Die jeweilige Piezoelementscheibe 2.1, 2.2 ist zumindest für den Zeitpunkt der Messung aktiv angesteuert. Die Werkzeugspitze 3 muss in diesem Fall über die erste Piezoelementscheibe 2.1 angetrieben sein, so dass die Schwingungsrichtung X gemäß Teildarstellung in Fig. 5a normal zum Zahn 6 verläuft. Aufgrund dieser relativen Position erfährt die Werkzeugspitze 3 und damit die erste Piezoelementscheibe 2.1 eine mechanische Dämpfung, die zu dem dargestellten Impedanzverlauf |Z|_{B}(f) unter Belastung führt. Unter der Belastung weist der Impedanzverlauf |Z| (f) in der Resonanz R1, R1' den Wert W1B auf, der größer ist als der Wert W1L im Leerlauf (gestrichelt dargestellt). Die Abweichung ist mit A1 gekennzeichnet. Aufgrund der Dämpfung kommt es zu einem leichten Abfall der Resonanzfrequenz von R1 auf R1'. Die Abweichung A1 beträgt in der Regel weniger als 500 Hz. Der Impedanzverlauf |Z|_{L}(f) im Leerlauf ist gestrichelt dargestellt.

In der Schwingungsrichtung Y erfolgt die Bewegung relativ zum Zahn 6 in tangentialer Richtung, so dass keine oder eine nur sehr geringe Dämpfung für die zweite Piezoelementscheibe 2.2 erfolgt. Die Messung der Dämpfung erfolgt durch kurzzeitigen Antrieb der zweiten Piezoelementscheibe 2.2.

Die zweite Piezoelementscheibe 2.2 weist ein Impedanzverlauf |Z|_{L}(f) entsprechend dem Leerlauf auf.

Erfindungsgemäß erfolgt ein Wechsel des Antriebs von der ersten Piezoelementscheibe 2.1 zur zweiten Piezoelementscheibe 2.2, damit die tangential zum Zahn 6 angeordnete Bewegungsrichtung X erzeugt wird.

Der Darstellung gemäß Fig. 5b liegt eine Dämpfung in der Schwingungsrichtung Y der Werkzeugspitze 3 zugrunde, während die Werkzeugspitze 3 in der Schwingungsrichtung X ungedämpft ist. Die Schwingungsrichtung Z verläuft in der Teildarstellung in Fig. 5b normal zum Zahn 6. In entsprechender Weise zeigt der Impedanzverlauf |Z|_{L}(f) der ersten Piezoelementscheibe 2.1 ein Leerlaufverhalten, wohingegen der Impedanzverlauf |Z|_{B}(f) der zweiten Piezoelementscheibe 2.2 einen Anstieg des Wertes W2L aufweist. Somit ergibt sich die Abweichung A2 zwischen dem Wert W2L und dem Wert W2B. Erfindungsgemäß erfolgt zwecks Antriebs ein Wechsel von der zweiten Piezoelementscheibe 2.2 zur ersten Piezoelementscheibe 2.1, damit die tangential zum Zahn 6 angeordnete Bewegungsrichtung X erzeugt wird.

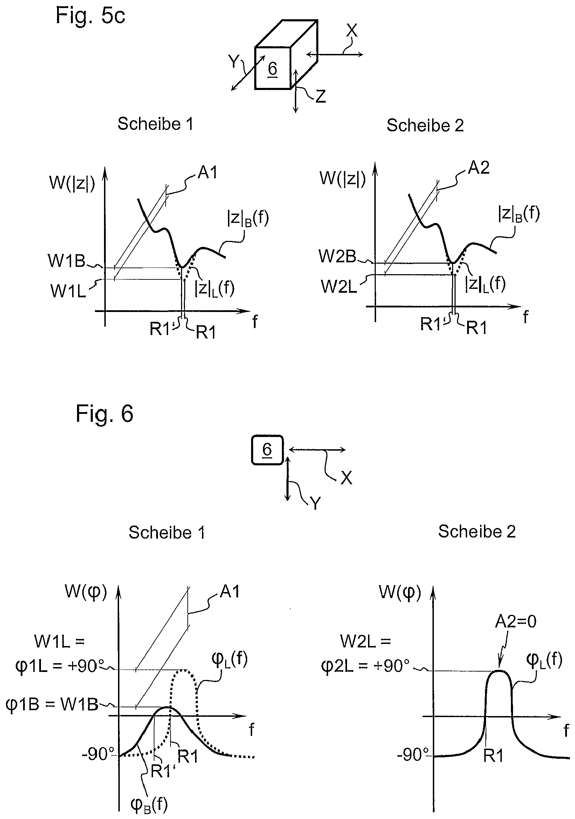

In der Darstellung gemäß Fig. 5c liegt sowohl in der Schwingungsrichtung X als auch in der Schwingungsrichtung Y eine Dämpfung der Werkzeugspitze 3 vor, während die Werkzeugspitze 3 in der Schwingungsrichtung Z ungedämpft ist. Sowohl die Schwingungsrichtung X als auch die die Schwingungsrichtung Y verlaufen in der Teildarstellung in Fig. 5b normal zum Zahn 6. Somit weisen sowohl der Impedanzverlauf |Z|_{L}(f) der ersten Piezoelementscheibe 2.1 als auch der Impedanzverlauf |Z|_{B}(f) der zweiten Piezoelementscheibe 2.2 einen Anstieg des Wertes W1L, W2L auf. Die Abweichung A1, A2 ergibt sich sowohl zwischen dem Wert W1L und dem Wert W1B als auch zwischen dem Wert W2L und dem Wert W2B. Erfindungsgemäß erfolgt ein Wechsel der Schwingungsrichtung X, Y in Richtung Z. Hierzu ist die Ansteuerung der Piezoelementscheiben 2.1, 2.2 symmetrisch zur Mittelachse Z notwendig, so dass eine rein axial verlaufende Schwingungsrichtung X erzeugt wird. Alternativ kann zusätzlich eine ungeteilte unidirektional arbeitende Piezoelementscheibe 2.1, 2.2 vorgesehen sein.

In der Darstellung gemäß Fig. 6 sind die Dämpfungsverhältnisse gemäß Fig. 5a gegeben, wobei der Verlauf des Wertes W(ϕ) für die Phase dargestellt ist, also der Phasenverlauf ϕ_{B}(f) unter Belastung und der Phasenverlauf ϕ_{L}(f) im Leerlauf. Aufgrund der relativen Position erfährt die Werkzeugspitze 3, und damit die erste Piezoelementscheibe 2.1, eine mechanische Dämpfung, die zu dem dargestellten Phasenverlauf ϕ_{B}(f) unter Belastung führt. Unter der Belastung weist die Phase ϕ den Maximalwert W1B auf, der kleiner ist als der Maximalwert W1L im Leerlauf. Die Abweichung ist mit A1 gekennzeichnet. Der Phasenverlauf ϕ_{L}(f) im Leerlauf ist gestrichelt dargestellt. Aufgrund der Dämpfung kommt es zu einem Abfall der Resonanzfrequenz von R1 auf R1'. In der Resonanz ist die Phase ϕ etwa gleich Null.

Durch Vergleich des Impedanz- bzw. Phasenverhaltens unter Last mit dem lastfreien Verhalten ist somit über die Mittel 4.1 eine Auswertung dahingehend möglich, inwieweit sich die Werkzeugspitze 3 in radialer bzw. normaler Richtung zum Zahn 6 bewegt. Sobald dies der Fall ist, wird die für den Antrieb angesteuerte Piezoelementscheibe 2.1, 2.2 bzw. die Schwingungsrichtung X, Y, Z gewechselt, so dass eine tangentiale Bewegung zum Zahn 6 erfolgt. In jedem Fall wird die Piezoelementscheibe 2.1, 2.2 angesteuert, deren Schwingungsrichtungen X, Y, Z den geringsten Anteil einer Richtung normal zur Zahnoberfläche aufweisen. Hierzu wird die Abweichung A1, A2 zwischen dem jeweiligen Wert W1B, W2B und dem zugehörigen Wert W1L, W2L ermittelt; entweder durch Differenzbildung oder durch Bildung des Quotienten Q1, Q2. Mit der Piezoelementscheibe 2.1, 2.2, die die kleinste Abweichung A1, A2 bzw. den kleinsten Quotienten Q1, Q2 aufweist, wird auch die Schwingungsrichtung X, Y erzeugt, die die geringste Dämpfung erfährt. Zwecks Ermittlung des Impedanz- bzw. Phasenverlaufs wird die jeweilige Piezoelementscheibe 2.1 - 2.4 zumindest kurzzeitig aktiv angesteuert.

Bei der tangentialen Bewegung, kann es sich neben der in Fig. 1 dargestellten geradlinigen Bewegung in Richtung X ebenfalls um eine überlagerte Bewegung in die Richtungen X/Z, X/Y oder Y/Z, also z. B. um eine plane Kreisbewegung, handeln. Weitere Überlagerungen der zur Verfügung stehenden Schwingungsrichtungen X, Y, Z sind ebenfalls vorgesehen.

Auflistung der ursprünglichen Erzeugnisansprüche:
1. Dentale Ultraschallvorrichtung (1) bestehend aus einem Handstück (5), aus einem eine Mittelachse Z aufweisenden Ultraschallantrieb (2) mit mindestens einer ersten Piezoelementscheibe (2.1) und mindestens einer zweiten Piezoelementscheibe (2.2), aus einer von dem Ultraschallantrieb (2) angetriebenen, mindestens- eine Resonanzfrequenz R1 für eine Schwingungsrichtung X rechtwinklig zur Mittelachse Z in einer Schwingungsebene S aufweisenden Werkzeugaufnahme (3.1) für eine Werkzeugspitze (3) und aus einer Regelungseinheit (4), die zur Ansteuerung mindestens einer der Piezoelementscheiben (2.1, 2.2) dient, wobei die Piezoelementscheiben (2.1, 2.2) mit Bezug zu der Mittelachse Z hintereinander angeordnet sind und die Piezoelementscheieiner Grenzebene E1, E2 gegenüberliegend angeordneten Piezoelementsegmenten (2.1a, 2.1b, 2.2a, 2.2b) besteht, wobei aus der Ansteuerung der ersten Piezoelementscheibe (2.1) die Schwingungsrichtung X erzeugbar ist, **dadurch gekennzeichnet,**
   - dass die zweite Piezoelementscheibe (2.2) so angeordnet ist, dass die Grenzebene E2 mit Bezug zur Umfangsrichtung zur Mittelachse Z verdreht zur Grenzebene E1 liegt, und
   - dass die jeweilige Piezoelementscheibe (2.1, 2.2) mittels der Regelungseinheit (4) getrennt ansteuerbar ist.
2. Dentale Ultraschallvorrichtung (1) nach Anspruch 1, dadurch gekennzeichnet, dass die Regelungseinheit (4) Mittel (4.1) aufweist, durch die zumindest ein von der mechanischen Dämpfung der aktiv angesteuerten Piezoelementscheibe (2.1, 2.2) abhängiger Wert W1B, W2B erfassbar ist, wobei der Wert W1B der Wert der ersten Piezoelementscheibe (2.1) und der Wert W2B der Wert der zweiten Piezoelementscheibe (2.2) ist und der Wert W1B, W2B ein Impedanzwert Z1B, Z2B oder ein Phasenwert ϕ1B, ϕ2B zwischen dem Strom I und der Spannung U unter Belastung ist.
3. Dentale Ultraschallvorrichtung (1) nach Anspruch 1, dadurch gekennzeichnet, dass die jeweilige Grenzebene E1, E2 parallel zu der Mittelachse Z angeordnet ist und die Grenzebene E1 mit der Grenzebene E2 einen Winkel α einschließt.
4. Dentale Ultraschallvorrichtung (1) nach Anspruch 2, dadurch gekennzeichnet, dass der Winkel α zwischen 5° und 90° groß ist.
5. Dentale Ultraschallvorrichtung (1) nach Anspruch 1, 2 oder 3 dadurch gekennzeichnet, dass die Mittel (4.1)
   a) zur Ermittlung oder Speicherung von zumindest einem Wert W1L, W2L zumindest einer Piezoelementscheibe (2.1, 2.2) im Leerlauf ausgebildet sind,
   b) zum Vergleich des Wertes W1B, W2B unter Belastung mit dem jeweiligen Wert W1L, W2L im Leerlauf ausgebildet sind und
   c) zur individuellen Ansteuerung beider Piezoelementscheiben (2.1, 2.2) ausgebildet sind.
6. Dentale Ultraschallvorrichtung (1) nach einem der vorhergehenden Ansprüche, dadurch gekennzeichnet, dass in Richtung der Mittelachse Z die Polarisierung der benachbarten Piezoelementsegmente (2.1a, 2.1b, 2.2a, 2.2b) oder die Polarität der elektrischen Ansteuerung der benachbarten Piezoelementsegmente (2.1a, 2.1b, 2.2a, 2.2b) entgegengesetzt ausgebildet ist.
7. Dentale Ultraschallvorrichtung (1) nach einem der vorhergehenden Ansprüche, dadurch gekennzeichnet, dass das jeweilige Piezoelementsegment (2.1a, 2.1b, 2.2a, 2.2b) teilkreisscheibenförmig oder rechteckförmig ausgebildet ist.
8. Dentale Ultraschallvorrichtung (1) nach einem der vorhergehenden Ansprüche, dadurch gekennzeichnet, dass das Handstück (5) und/oder die Regelungseinheit (4) ein Signalmittel (4.2) aufweist, über das ein akustisches, optisches und/oder taktiles Signal erzeugbar ist.

### Bezugszeichenliste

- 1: Ultraschallvorrichtung
- 2: Ultraschallantrieb
- 2.1: erste Piezoelementscheibe
- 2.1a: Piezoelementsegment
- 2.1b: Piezoelementsegment
- 2.2: zweite Piezoelementscheibe
- 2.2a: Piezoelementsegment
- 2.2b: Piezoelementsegment
- 2.3: Piezoelementscheibe
- 2.3a: Piezoelementsegment
- 2.3b: Piezoelementsegment
- 2.4: Piezoelementscheibe
- 2.4a: Piezoelementsegment
- 2.4b: Piezoelementsegment
- 2.5: Kontaktleitung
- 3: Werkzeugspitze
- 3.1: Werkzeugaufnahme
- 4: Regelungseinheit
- 4.1: Mittel
- 4.2: Signalmittel
- 5: Handstück
- 5.1: Versorgungsleitung
- 6: Zahn
- 7.1a: Kontaktelement
- 7.1b: Kontaktelement
- 7.1c: Kontaktelement
- 7.1d: Kontaktelement
- 7.2a: Kontaktelement
- 7.2b: Kontaktelement
- 7.2c: Kontaktelement
- 7.2d: Kontaktelement
- 7.3a: Kontaktelement
- 7.3c: Kontaktelement
- 7.4a: Kontaktelement
- 7.4c: Kontaktelement
- 8.1: Schwungmasse
- 8.2: Endmasse
- 9.1: Isolierscheibe
- 9.2: Isolierscheibe
- 9.3: Isolierscheibe
- 9.4: Isolierscheibe
- 9.5: Isolierscheibe
- A1: Abweichung bezüglich erste Scheibe
- A2: Abweichung bezüglich zweite Scheibe
- α: Winkel
- E1: Grenzebene
- E2: Grenzebene
- f: Anregungsfrequenz
- ϕ: Phase
- ϕ(f): Phasenverlauf
- ϕ_{L}(f): Phasenverlauf im Leerlauf
- ϕ_{B}(f): Phasenverlauf unter Last (Belastung)
- ϕ1L: Phase für erste Scheibe im Leerlauf
- ϕ1B: Phase für erste Scheibe unter Belastung
- ϕ2L: Phase für zweite Scheibe im Leerlauf
- ϕ2B: Phase für zweite Scheibe unter Belastung
- I: Strom
- Q1: Quotient für erste Scheibe
- Q2: Quotient für zweite Scheibe
- R1: Resonanzfrequenz
- R1': Resonanzfrequenz
- S: Schwingungsebene
- U: Spannung
- W(ϕ): Wert
- W(|Z|): Wert der Impedanz
- W1B: Wert für erste Scheibe unter Belastung
- W1L: Wert für erste Scheibe im Leerlauf
- W2B: Wert für zweite Scheibe unter Belastung
- W2L: Wert für zweite Scheibe im Leerlauf
- X: Schwingungsrichtung
- Y: Schwingungsrichtung
- Z: Mittelachse, Schwingungsrichtung
- |Z|(f): Impedanzverlauf, Impedanz
- |Z|_{L}(f): Impedanzverlauf
- |Z|_{B}(f): Impedanzverlauf
- Z1B: Impedanz unter Belastung (Last)
- Z2B: Impedanz unter Belastung (Last)
- Z1L: Impedanz im Leerlauf (ohne Last)
- Z2L: Impedanz im Leerlauf (ohne Last)

## Patentansprüche

1. Verfahren zum Betrieb einer dentalen Ultraschallvorrichtung (1), **dadurch gekennzeichnet, dass**
a) ein von der mechanischen Dämpfung abhängiger Wert W1B, W2B einer aktiv angesteuerten Piezoelementscheibe (2.1, 2.2) zumindest bei der Resonanzfrequenz R1 erfasst wird,
b) zumindest der Wert W1B, W2B mit zumindest einem für die Resonanzfrequenz R1 und die entsprechende Piezoelementscheibe (2.1, 2.2) im Leerlauf ermittelten Wert W1L, W1L verglichen wird,
c) diejenige Piezoelementscheibe (2.1, 2.2) aktiv angesteuert wird, bei der die Abweichung A1, A2 zwischen dem Wert W1B, W2B der jeweiligen Piezoelementscheibe (2.1, 2.2) unter Belastung und dem Wert W1L, W2L kleiner ist.

2. Verfahren nach Anspruch 1, **dadurch gekennzeichnet, dass** der Wert W1B, W2B ein Impedanzwert Z1B, Z2B oder ein Phasenwert ϕ1B, ϕ2B zwischen dem Strom I und der Spannung U unter Belastung ist und der Wert W1L, W2L ein Impedanzwert Z1L, Z2L oder ein Phasenwert ϕ1L, ϕ2L zwischen dem Strom I und der Spannung U im Leerlauf ist.

3. Verfahren nach Anspruch 2, **dadurch gekennzeichnet, dass** in Schritt c) zwecks Bewertung der Abweichung A1, A2 ein Quotient Q1, Q2 von dem Impedanzwert Z1B, Z2B zu dem entsprechenden Impedanzwert Z1L, Z2L oder von dem Phasenwert ϕ1L, ϕ2L zu dem entsprechenden Phasenwert ϕ1B, ϕ2B berechnet wird und diejenige Piezoelementscheibe (2.1, 2.2) aktiv angesteuert wird, bei der der Quotient Q1, Q2 kleiner ist.

4. Verfahren nach Anspruch 1, 2 oder 3, **dadurch gekennzeichnet, dass** bei Überschreiten eines Schwellenwertes für die Abweichung A1, A2 oder für den Quotienten Q1, Q2 diejenige Piezoelementscheibe (2.1, 2.2) aktiv angesteuert wird, deren Grenzebene E1, E2 um den Winkel α versetzt zu der aktuell aktiv angesteuerten Piezoelementscheibe (2.1, 2.2) angeordnet ist.

5. Verfahren nach einem der Ansprüche 1 bis 4, **dadurch gekennzeichnet, dass** der Vergleich nach Schritt b) und/oder das Vergleichen der Abweichung A1, A2 und das Aktivieren der Ansteuerung nach Schritt c) mit einer Frequenz zwischen 10 Hz und 500 Hz erfolgt.

6. Verfahren nach einem der Ansprüche 1 bis 5, **dadurch gekennzeichnet, dass** die Regelungseinheit (4) ein Warnsignal erzeugt, wenn die mechanische Dämpfung der Werkzeugspitze (3) einen kritischen Wert oder der Quotient Q1, Q2 einen Wert von 2 oder 3 überschreitet.

7. Verfahren nach einem der Ansprüche 1 bis 6, **dadurch gekennzeichnet, dass** eine Verschiebung der Resonanzfrequenz R1 im Leerlauf und einer Resonanzfrequenz R1' unter Belastung bei Ermittlung des Wertes W1B, W2B und des Wertes W1L, W2L berücksichtigt wird.
